# FASCICULE DE BREVET EUROPEEN

(11) **EP 0 604 249 B1**
(45) Date de publication et mention de la délivrance du brevet: **16.04.1997**
(21) Numéro de dépôt: 93402841.6
(22) Date de dépôt: 23.11.1993
(51) Int. Cl.: A61K 7/42, A61K 7/48

(54) **Compositions à base de dihydroxyacétone et leur utilisation cosmétique**
Mittel enthaltend Dihydroxyaceton und ihre kosmetische Verwendung
Compositions containing dihydroxyacetone and their cosmetic use

(30) Priorité: 24.11.1992 FR 9214105
(43) Date de publication de la demande: 29.06.1994
(73) Titulaire: L'OREAL, 75008 Paris (FR)
(72) Inventeur: Hansenne, Isabelle, F-75017 Paris (FR); Forestier, Serge, F-77410 Claye-Souilly (FR); Petitdemange, Delphine, F-92700 Colombes (FR)
(74) Mandataire: Casalonga, Axel

(56) Documents cités:
- EP-A- 0 406 042
- EP-A- 0 503 853
- EP-A- 0 576 189
- WO-A-92/17159
- US-A- 5 232 688

## Description

L'invention est relative à de nouvelles compositions cosmétiques à base de dihydroxyacétone ou DHA et au procédé de coloration de la peau les mettant en oeuvre.

La dihydroxyacétone ou DHA est connue depuis de nombreuses années dans son application à la coloration de la peau.

Les compositions contenant de la DHA présentent cependant un certain nombre de problèmes suivant la nature du milieu dans lequel la DHA est formulée. (Voir Bobin et Coll Farmaco Ed Pr vol 38 fasicule 11-1983 p.415-423). On sait ainsi que différents facteurs peuvent influencer la conservation de la DHA dans une composition cosmétique et notamment le pH, la température de stockage, la nature réductrice ou oxydante du milieu, le solvant.

Les compositions proposées jusqu'à présent à base de DHA étaient essentiellement épaissies par des dérivés de cellulose tels que par exemple par l'hydroxypropylcellulose ou l'hydroxyéthylcellulose. Ces compositions présentent cependant l'inconvénient de poser des problèmes de conservation au cours du temps. C'est ainsi que l'on a noté après un stockage de deux mois à 45°C une chute de viscosité de ces compositions et un jaunissement non souhaitable. Par ailleurs, au niveau cosmétique les compositions appliquées sur la peau donnent généralement un caractère collant.

Le document WO-A-9217159 décrit une composition de coloration de la peau contenant de la dihydroxyacetone et un copolymère d'une acide carboxylique.

La demanderesse a découvert qu'en utilisant dans des compositions cosmétiques la DHA avec un copolymère réticulé d'acrylamide/acide 2-acrylamido 2-méthylpropane sulfonique, il était possible de préparer des compositions de coloration de la peau particulièrement stables au stockage. En particulier, on ne constate pas, après stockage dans les conditions précitées une chute de viscosité et le jaunissement de la composition. De plus l'utilisation de ces copolymères conduit à des compositions épaissies, présentant de bonnes propriétés cosmétiques dans la mesure où la composition confère à la peau une douceur sans présenter de sensation de collant. Enfin, la composition conforme à l'invention permet d'obtenir une coloration de la peau après application, similaire au bronzage naturel, plus intense et ayant une meilleure tenue à l'eau.

L'invention a donc pour objet une composition cosmétique de coloration de la peau sous forme d'émulsion huile dans eau ou de dispersion vésiculaire à base de dihydroxyacétone et d'un copolymère réticulé d'acrylamide/acide 2-acrylamido 2-méthylpropane sulfonique neutralisé partiellement ou en totalité.

Un autre objet de l'invention est constitué par le procédé de coloratiori de la peau mettant en oeuvre une telle composition.

D'autres objets de l'invention apparaîtront à la lecture de la description et des exemples qui suivent.

La composition de coloration de la peau conforme à l'invention est essentiellement caractérisée par le fait qu'elle contient dans un milieu cosmétiquement acceptable au moins de la dihydroxyacétone et à titre d'agent épaississant au moins un copolymère réticulé d'acrylamide/acide 2-acrylamido 2-méthylpropane sulfonique neutralisé partiellement ou en totalité.

La dihydroxyacétone ou DHA est présente dans les compositions conformes à l'invention dans des proportions suffisantes pour conférer à la peau, après application, une coloration similaire à la coloration obtenue à la suite d'un bronzage naturel. Elle est généralement présente dans des proportions comprises entre 0,5 et 10 % en poids par rapport au poids total de la composition et de préférence entre 1 et 7 % en poids.

Le copolymère réticulé d'acrylamide/acide 2-acrylamido 2-méthylpropane sulfonique neutralisé partiellement ou totalement est plus particulièrement un copolymère réticulé par un composé à poly insaturation oléfinique tel que le tétraallyloxyéthane, l'allylsucrose, l'allylpentaerythritol ou le méthylène bis-acrylamide, partiellement ou totalement neutralisé par un agent de neutralisation tel que la soude, la potasse ou une amine tertiaire telle que la triéthanolamine. Ces copolymères peuvent être préparés en copolymérisant l'acrylamide et le 2-acrylamido 2-méthylpropane sulfonate de sodium par voie radicalaire au moyen d'agents initiateurs du type azo-bis-isobutyronitrile, et suivi par une précipitation dans un alcool tel que le tertio-butanol.

Les copolymères d'acrylamide et de 2-acrylamido 2-méthylpropane sulfonate plus particulièrement préférés conformément à l'invention sont ceux obtenus par copolymérisation de 70 à 55 % en mole d'acrylamide et de 30 à 45 % en mole de 2-acrylamido 2-méthylpropane sulfonate de sodium. L'agent de réticulation est utilisé dans des concentrations de 10⁻⁴ à 4.10⁻⁴ mole par mole du mélange des monomères. Un copolymère plus particulièrement préféré est le produit commercialisé par la société SEPPIC sous la dénomination Sepigel 305 sous la forme d'une émulsion huile dans eau contenant de 35 à 40 % en poids de ce copolymère de 15 à 25 % en poids d'un mélange d'hydrocarbures isoparaffiniques en C₁₂-C₁₃, de 3 à 8 % en poids de lauryléther de polyéthylèneglycol à 7 moles d'oxyde d'éthylène et d'eau.

Le copolymère réticulé d'acrylamide/acide 2-acrylamido 2-méthylpropane sulfonique neutralisé totalement ou partiellement est utilisé dans des quantités suffisantes pour épaissir la composition, qui doit pouvoir être appliquée sur la peau sans couler. Ce copolymère est utilisé de préférence dans des proportions comprises entre 0,5 et 5 % en poids en matière active par rapport au poids total de la composition et de préférence entre 0,75 et 3 % en poids en matière active par rapport au poids total de la composition.

Les compositions conformes à l'invention se présentent sous forme d'émulsion huile dans eau ou de dispersion vésiculaire.

Le milieu utilisé dans ces compositions contient généralement de l'eau, un mélange d'eau et d'un ou plusieurs solvants organiques, ou un solvant ou un mélange de solvants organiques cosmétiquement acceptables. Ce milieu contient également sous une forme de réalisation préférentielle, des corps gras et/ou des silicones cosmétiquement acceptables.

Les solvants sont en particulier choisis parmi les alcools inférieurs en C₁-C₄ tels que l'alcool éthylique, l'alcool isopropylique; les monoalcools saturés à longue chaîne ayant 10 à 18 atomes de carbone; les polyols ayant 2 à 8 atomes de carbone tels que le glycérol, les alkylèneglycols comme l'éthylèneglycol, le propylèneglycol, le diéthylèneglycol; les esters de mono ou diacides ayant de préférence 4 à 22 atomes de carbone et d'alcools saturés linéaires ou ramifiés ayant de 1 à 22 atomes de carbone. On peut citer parmi eux l'adipate de diisopropyle ou de di (2-éthylhexyle), le maléate de di (2-éthylhexyle), les benzoates d'alcools en C₁₂-C₁₅ et le néopentanoate d'isoarachidyle.

Ces solvants sont généralement utilisés dans le milieu aqueux dans des proportions comprises entre 0,5 et 75 % en poids et en particulier entre 2 et 50 % en poids par rapport au poids total de la composition globale.

Parmi les corps gras, on peut citer les huiles et cires d'origine minérale, animale, végétale ou synthétique, telles que l'huile de jojoba, de ricin, de vaseline; les huiles de synthèse sont notamment :
- soit des isoparaffines de viscosité inférieure à 0,5 Pa.s répondant à la formule 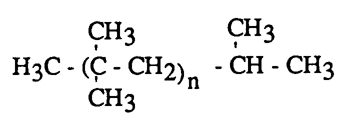 , dans laquelle,
   n est compris entre 2 et 16 et leurs mélanges avec des huiles de structure identique, dans laquelle n est supérieur à 18 et de préférence compris entre 18 et 40; de telles huiles sont vendues par la société PRESPERSE INC. sous les dénominations PERMETHYL 99A, 101A, 102A, 104A, 106A,
   ou par la société ICI sous la dénomination ARLAMOL HD.
On peut citer, aussi les ISOPARS vendus par la société EXXON INC.
- soit des poly 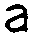 -oléfines de type polydécène hydrogénées ou non; de tels produits sont vendus par la société ETHYL CORPORATION sous la dénomination ETHYLFLO ; on cite aussi les polyisobutylènes hydrogénés ou non.

Les corps gras comprennent également les acides gras; les alcools gras tels que l'alcool laurylique, cétylique, myristylique, stéarylique, palmitylique, oléique ainsi que le 2-octyldodécanol, les esters d'acides gras tels que le monostéarate de glycérol, le monostéarate de polyéthylèneglycol, les triglycérides d'acides gras en C₆-C₁₈ tels que les triglycérides d'acide caprylique/caprique.

Les silicones utilisables dans les compositions conformes à l'invention sont des organopolysiloxanes tels que les huiles d'organopolysiloxanes, des solutions organiques de gommes et/ou de résines d'organosiloxanes.

Parmi ces silicones on peut citer les silicones volatiles ayant un point d'ébullition compris entre 60°C et 260°C, telles que les silicones cycliques de 3 à 7 atomes de silicium, les silicones volatiles linéaires ayant de 2 à 9 atomes de silicium et ayant une viscosité inférieure ou égale à 5 x 10⁻⁶m²/s à 25°C.

Les silicones non volatiles sont constituées principalement par les polyalkylsiloxanes, les polyarylsiloxanes, les polyalkylarylsiloxanes, les copolymères polyéthersiloxanes modifiés ou non, les gommes et résines de silicone et les polysiloxanes organo modifiés, ainsi que leurs mélanges.

Parmi les polyalkylsiloxanes, on peut citer
- les polydiméthylsiloxanes linéaires de viscosité supérieure à 10⁻¹ m²/s, pouvant comporter des groupements terminaux triméthyl silyle, trihydroxysilyle ou hydroxydiméthylsilyle.
- les polyalkylarylsiloxanes, tels que les polydiméthylphénylsiloxanes, les polyméthyldiphénylsiloxanes linéaires et/ou ramifiés de viscosité de 10⁻⁵ à 5. 10⁻²m²/s à 25°C;
- les polyalkyl (C₁₀-C₃₀) siloxanes à groupements terminaux triméthylsilyle.
- les copolymères de polyéthersiloxanes modifiés ou non sont choisis notamment parmi les copolymères d'oxyde d'éthylène et/ou de propylène avec un di-organosiloxane tels que les diméthicone copolyols,
- les gommes de silicone sont entre autre des polydiorganosiloxanes de masse moléculaire comprise entre 200 000 et 1 000 000, utilisés seuls ou en mélange dans un solvant choisi parmi les silicones volatiles, les huiles de polydiméthylsiloxanes, les huiles de polyphénylméthylsiloxanes, les isoparaffines, le dodécane, le tridécane, le tétradécane ou leurs mélanges;
- les résines d'organopolysiloxanes utilisables conformément à l'invention sont des systèmes siloxaniques réticulés renfermant des unités R₂ SiO₂/₂, R Si O₃/₂, Si O₄/₂ dans lesquelles R représente un groupement hydrocarboné possédant de 1 à 6 atomes de carbone ou un groupement phényle. Le groupement hydrocarboné désigne de préférence un radical alkyle en C₁-C₄.
- les silicones organomodifiées sont choisies notamment parmi les silicones précitées comportant dans leur structure générale un ou plusieurs groupements organo fonctionnels directement fixés sur la chaîne siloxanique ou fixés par l'intermédiaire d'un radical hydrocarboné. On peut citer à cet effet les silicones comportant des groupements perfluorés tels que des groupements trifluoroalkyles; des groupements hydroxylés comme les polyorganosiloxanes à fonction hydroxyalkyle; des groupements alcoxylés; des groupements acyloxyalkyle;

Les polyorganosiloxanes particulièrement préférés sont choisis parmi les silicones non volatiles du type polyalkylsiloxanes linéaires à groupements terminaux triméthylsilyle tels que
- les huiles SILBIONE des séries 70 047 et 47 tels que l'huile 47V 500 000 commercialisée par la société Rhône Poulenc;
- des mélanges d'organopolysiloxanes et de silicone cyclique tels que le produit Q2 1 401 de la société Dow Corning ou SF 1214 SILICONE FLUID de la société Général Electric.
- les fluorosilicones de type polyalkylsiloxane à groupements terminaux triméthylsilyle et substitués sur la chaîne par des groupements trifluoropropyle tels que la fluorosilicone vendue par la société SHIN ETSU sous la dénomination X 22-821.
- des silicones volatiles linéaires ou cycliques et plus particulièrement le décaméthylcyclopentasiloxane.

Les polyorganosiloxanes sont généralement utilisés dans les compositions dans des proportions comprises entre 0,5 et 50 % en poids et de préférence entre 1 et 30 % en poids par rapport au poids total de la composition.

Selon une forme de réalisation préférée de l'invention, la composition contient des filtres solaires liposolubles et hydrosolubles UV-B et/ou UV-A.

Ces filtres solaires sont choisis en particulier parmi les dérivés cinnamiques tels que par exemple le p-méthoxycinnamate de 2-éthylhexyle; les dérivés salicyliques comme par exemple le salicylate de 2-éthylhexyle; les dérivés du camphre comme par exemple le 4-méthylbenzylidène camphre, l'acide benzène 1,4[di(3-méthylidène 10-camphosulfonique)]; les dérivés du benzimidazole tels que l'acide 2-phénylbenzimidazole 5-sulfonique; les dérivés de triazine tels que la 2,4,6-tris [p-(2'-éthylhexyl-1'-oxycarbonyl)anilino] 1,3,5-triazine; les dérivés de la benzophénone tels que la 2-hydroxy 4-méthoxy, benzophénone; les dérivés du dibenzoylméthane tels que le 4 tert-butyl 4'-méthoxydibenzoylméthane; les dérivés de 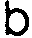 , 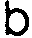 -diphénylacrylate tels que le 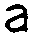 -cyano- 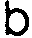 , 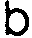 -diphénylacrylate de 2-éthylhexyle.

Ces filtres solaires sont utilisés dans des proportions comprises entre 0,01 et 15 % en poids par rapport au poids total de la composition.

Le pH des compositions conformes à l'invention est généralement compris entre 4 et 10 et de préférence entre 5 et 7,5.

Dans le cas d'émulsion, on peut utiliser des émulsionnants bien connus en cosmétique et notamment l'alcool cétylique ou l'alcool oléique oxyéthylèné avec 5 à 30 moles d'oxyde d'éthylène; l'oléate de sorbitan oxyéthylèné avec 10 à 40 moles d'oxyde d'éthylène, des mélanges de monostéarate de glycérol et de stéarate de polyéthylèneglycol à 50 à 100 moles d'oxyde d'éthylène.

Les compositions peuvent également se présenter sous forme de dispersions vésiculaires de lipides amphiphiles ioniques ou non ioniques préparés suivant des procédés connus. On peut par exemple faire gonfler les lipides dans une solution aqueuse pour former des sphérules dispersées dans le milieu aqueux comme décrit dans l'article BANGHAM, STANDISH et WATKINS, J.mol. Biol., 13, 238 (1965) ou dans les brevets FR-2.315.991 et 2.416.008, on trouvera la description des divers modes de préparation dans "Les liposomes en biologie cellulaire et pharmacologie" Edition INSERM, John Libbery - Eurotext, 1987, pages 6 à 18.

Les compositions conformes à l'invention peuvent également contenir tout autre adjuvant utilisé habituellement dans des compositions cosmétiques pour la peau et plus particulièrement des agents tensio-actifs de préférence des agents tensio-actifs non ioniques, des agents hydratants, adoucissants, des vitamines, des antioxydants, des opacifiants, des colorants des parfums et/ou des agents conservateurs.

Les solvants, corps gras ou adjuvants utilisés dans des compositions conformes à l'invention ne doivent comporter ni groupement amine primaire ou secondaire, ni groupement oxydant susceptible d'interférer avec la DHA.

Ces compositions peuvent également contenir ou être utilisées conjointement avec des substances pouvant avoir un effet de nuançage ou de coloration supplémentaire de la peau en association avec la dihydroxyacétone. On peut utiliser à cet effet en particulier des hydroxyindoles.

Le procédé de coloration de la peau qui est un autre objet de l'invention est essentiellement caractérisé par le fait que l'on applique sur la peau, une composition telle que définie ci-dessus dans des quantités suffisantes pour conférer à la peau une coloration similaire au bronzage naturel.

Les exemples qui suivent sont destinés à illustrer l'invention.

| **Exemple 1 : Gel autobronzant** | |
|---|---|
| DHA | 5 g |
| Mélange de diméthiconol (13 %), d'octaméthyl-cyclotétrasiloxane et de décaméthylcyclopentasiloxane (87 %) vendu sous la dénomination DC Q2 1401 par la société Dow Corning | 5 g |
| Décaméthylcyclopentasiloxane (DC 245 Fluid de Dow Corning) | 5 g |
| Emulsion huile dans eau de copolymère réticulé d'acrylamide/2-acrylamido 2-méthylpropane sulfonate de sodium vendue sous la dénomination SEPIGEL 305 par la société SEPPIC. | 2 g MA |
| Conservateur | Qs |
| Parfum | Qs |
| Eau | Qsp 100 g |

| **Exemple 2 : Gel crème autobronzant** | |
|---|---|
| DHA | 3 g |
| Huile de Jojoba | 10 g |
| Acide benzène 1,4-[di(3-méthylidène 10-camphosulfonique)] | 1 g MA |
| Triéthanolamine | 0,6 g |
| Emulsion huile dans eau de copolymère réticulé d'acrylamide/2-acrylamido 2-méthylpropane sulfonate de sodium vendue sous la dénomination SEPIGEL 305 par la société SEPPIC. | 2, 5 g MA |
| Conservateur | Qs |
| Parfum | Qs |
| Eau | Qsp 100 g |

| **Exemple 3 : Emulsion autobronzante** | |
|---|---|
| Alcool cétylique oxyéthyléné à 10 moles d'oxyde d'éthylène vendu sous la dénomination Brij 56 par la société ICI | 5 g |
| Alcool cétylique | 2 g |
| Huile de vaseline | 10 g |
| Para-méthoxy cinnamate de 2-éthyl hexyle vendu sous la dénomination Parsol MCX | 3 g |
| Décaméthylcyclopentasiloxane vendu sousla dénomination DC 245 Fluid par la société Dow Corning | 5 g |
| DHA | 5 g |
| Emulsion huile dans eau de copolymère réticulé d'acrylamide/2-acrylamido 2-méthylpropane sulfonate de sodium vendue sous la dénomination SEPIGEL 305 par la société SEPPIC. | 1 g MA |
| Conservateur | Qs |
| Parfum | Qs |
| Eau | Qsp 100 g |

| **Exemple 4 : Emulsion autobronzante :** | |
|---|---|
| Dihydroxyacétone | 3,5 g |
| Emulsion H/E de copolymère réticulé acrylamide /2-acrylamido 2-méthyl propane sulfonate de sodium vendue par la société SEPPIC sous la dénomination SEPIGEL 305 | 1,6 g MA |
| Mélange de diméthiconol (13 %), d'octaméthylcyclotétrasiloxane et de décaméthylcyclopentasiloxane (87 %) vendu sous la dénomination Q2-1401 par la société Dow Corning | 10 g |
| Huile de vaseline | 10 g |
| Mélange de stéarate de glycérol et de stéarate de polyéthylène glycol à 100 moles d'oxyde d'éthylène vendu sous la dénomination ARLACEL 165 par la société ICI | 2 g |
| Conservateur, parfum | qs |
| Eau | qsp 100 g |

## Revendications

1. Composition cosmétique de coloration de la peau sous forme d'émulsion huile dans eau ou de dispersion vésiculaire caractérisée par le fait qu'elle contient dans un milieu cosmétiquement acceptable, au moins de la dihydroxyacétone et au moins un agent épaississant constitué par un copolymère réticulé d'acrylamide/acide 2-acrylamido 2-méthylpropane sulfonique neutralisé partiellement ou totalement.

2. Composition selon la revendication 1, caractérisée par le fait que la dihydroxyacétone est utilisée dans des proportions comprises entre 0,5 et 10 % en poids par rapport au poids total de la composition de préférence entre 1 et 7 % en poids.

3. Composition selon la revendication 1 ou 2, caractérisée par le fait que le copolymère réticulé d'acrylamide/acide2-acrylamido 2-méthylpropane sulfonique est un copolymère réticulé par un composé à poly insaturation oléfinique, partiellement ou totalement neutralisé par un agent de neutralisation choisi parmi la soude, la potasse, ou une amine tertiaire.

4. Composition selon l'une quelconque des revendications 1 à 3, caractérisée par le fait que le copolymère d'acrylamide et de 2-acrylamido 2-méthylpropane sulfonate est obtenu par copolymérisation de 70 à 55 % en mole d'acrylamide, et de 30 à 45 % en mole de 2-acrylamido 2-méthylpropane sulfonate de sodium et est réticulé par un composé à poly insaturation oléfinique à raison de 10⁻⁴ à 4.10⁻⁴ mole par mole du mélange des monomères.

5. Composition selon l'une quelconque des revendications 1 à 4, caractérisée par le fait que le copolymère réticulé d'acrylamide/acide 2-acrylamido 2-méthylpropane sulfonique est dans une émulsion H/E contenant 35 à 40 % en poids de copolymère, de 15 à 25 % du poids d'un mélange d'hydrocarbures isoparaffiniques en C₁₂-C₁₃, de 3 à 8 % en poids de lauryléther de polyéthylène glycol à 7 moles d'oxyde d'éthylène et d'eau.

6. Composition selon l'une quelconque des revendications 1 à 5, caractérisée par le fait que le copolymère d'acrylamide et d'acide 2-acrylamido 2-méthylpropane sulfonique neutralisé, partiellement ou totalement est présent dans des proportions comprises entre 0,5 et 5 % de matière active par rapport au poids total de la composition, de préférence de 0,75 à 3 % en poids de matière active par rapport au poids total de la composition.

7. Composition selon l'une quelconque des revendications 1 à 6, caractérisée par le fait qu'elle contient également des filtres solaires liposolubles et/ou hydrosolubles UV-B et/ou UV-A.

8. Composition selon la revendication 7, caractérisée par le fait que les filtres solaires sont choisis parmi les dérivés cinnamiques, les salicylates, les dérivés du benzylidène camphre, les dérivés du benzimidazole, les dérivés de triazine, les dérivés de la benzophénone, les dérivés du dibenzoylméthane ou les dérivés de 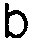 , 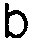 -diphénylacrylate.

9. Composition selon l'une quelconque des revendications 1 à 8, caractérisée par le fait que le milieu cosmétiquement acceptable comprend de l'eau, un mélange d'eau et d'un ou plusieurs solvants organiques, un solvant ou un mélange de solvants organiques cosmétiquement acceptables.

10. Composition selon l'une quelconque des revendications 1 à 9, caractérisée par le fait que la composition contient des corps gras choisis parmi les huiles et les cires d'origine minérale, animale, végétale ou synthétique, des acides gras, les alcools gras, les triglycérides d'acides gras en C₆-C₁₈, les esters de mono ou diacides.

11. Composition selon l'une quelconque des revendications 1 à 10, caractérisée par le fait quelle contient également un organo polysiloxane.

12. Composition selon la revendication 10, caractérisée par fait que l'organopolysiloxane est une silicone volatile ayant un point d'ébullition compris entre 60 et 260°C ou bien une silicone non volatile choisie parmi les polyalkylsiloxanes, les polyarylsiloxanes, les polyarylalkylsiloxanes, les copolymères polyéthersiloxanes modifiés ou non, les gommes et résines de silicone, les polysiloxanes organomodifiés ainsi que leurs mélanges.

13. Procédé de coloration de la peau pour lui conférer un bronzage similaire au bronzage naturel caractérisé par le fait que l'on applique sur la peau au moins une composition telle que définie dans l'une quelconque des revendications 1 à 12.

## Claims

1. Cosmetic composition for colouring the skin, in the form of an oil-in-water emulsion or a vesicular dispersion, characterized in that it contains at least dihydroxyacetone and at least one thickening agent, consisting of a partially or completely neutralized, crosslinked copolymer of acrylamide and 2-acrylamido-2-methylpropanesulphonic acid, in a cosmetically acceptable medium.

2. Composition according to Claim 1, characterized in that dihydroxyacetone is used in proportions of between 0.5 and 10% by weight relative to the total weight of the composition, and preferably between 1 and 7% by weight.

3. Composition according to Claim 1 or 2, characterized in that the crosslinked copolymer of acrylamide and 2-acrylamido-2-methylpropanesulphonic acid is a copolymer crosslinked with a compound containing olefinic polyunsaturation, partially or completely neutralized with a neutralizing agent chosen from sodium hydroxide, potassium hydroxide and a tertiary amine.

4. Composition according to any one of Claims 1 to 3, characterized in that the copolymer of acrylamide and 2-acrylamido-2-methylpropanesulphonate is obtained by copolymerization of 70 to 55 mol% of acrylamide and 30 to 45 mol% of sodium 2-acrylamido-2-methylpropanesulphonate, and is crosslinked with a compound containing olefinic polyunsaturation in the proportion of 10⁻⁴ to 4 × 10⁻⁴ mol per mole of the mixture of monomers.

5. Composition according to any one of Claims 1 to 4, characterized in that the crosslinked copolymer of acrylamide and 2-acrylamido-2-methylpropanesulphonic acid is in an O/W emulsion containing 35 to 40% by weight of copolymer, from 15 to 25% of the weight of a mixture of C₁₂-C₁₃ isoparaffinic hydrocarbons, from 3 to 8% by weight of polyethylene glycol lauryl ether containing 7 mol of ethylene oxide and water.

6. Composition according to any one of Claims 1 to 5, characterized in that the partially or completely neutralized copolymer of acrylamide and 2-acrylamido-2-methylpropanesulphonic acid is present in proportions of between 0.5 and 5% of active substance relative to the total weight of the composition, and preferably from 0.75 to 3% by weight of active substance relative to the total weight of the composition.

7. Composition according to any one of Claims 1 to 6, characterized in that it also contains fat-soluble and/or water soluble UV-B and UV-A sunscreen agents.

8. Composition according to Claim 7, characterized in that the sunscreen agents are chosen from cinnamic derivatives, salicylates, benzylidenecamphor derivatives, benzimidazole derivatives, triazine derivatives, benzophenone derivatives, dibenzoylmethane derivatives and b,b-diphenylacrylate [sic] derivatives.

9. Composition according to any one of Claims 1 to 8, characterized in that the cosmetically acceptable medium comprises water, a mixture of water and one or more organic solvents or a cosmetically acceptable organic solvent or mixture of solvents.

10. Composition according to any one of Claims 1 to 9, characterized in that the composition contains fats chosen from oils and waxes of mineral, animal, vegetable or synthetic origin, fatty acids, fatty alcohols, C₆-C₁₈ fatty acid triglycerides and esters of mono- or dibasic acids.

11. Composition according to any one of Claims 1 to 10, characterized in that it also contains an organopolysiloxane.

12. Composition according to Claim 10, characterized in that the organopolysiloxane is a volatile silicone having a boiling point of between 60 and 260°C, or alternatively a non-volatile silicone chosen from polyalkylsiloxanes, polyarylsiloxanes, polyarylalkylsiloxanes, polyethersiloxane copolymers, modified or otherwise, silicone gums and resins and organo-modified polysiloxanes, as well as mixtures thereof.

13. Process for colouring the skin in order to impart thereto a tan similar to a natural tan, characterized in that at least one composition as defined in any one of Claims 1 to 12 is applied to the skin.

## Patentansprüche

1. Kosmetische Zusammensetzung zur Färbung der Haut in Form einer Öl-in-Wasser-Emulsion oder einer bläschenartigen Dispersion,
dadurch **gekennzeichnet**, daß
sie in einem kosmetisch geeigneten Medium mindestens Dihydroxyaceton und mindestens ein Verdickungsmittel enthält, das aus einem vernetzten Acrylamid/2-Acrylamido-2-methylpropansulfonsäure-Copolymer zusammengesetzt ist, welches teilweise oder ganz neutralisiert ist.

2. Zusammensetzung gemäß Anspruch 1,
dadurch **gekennzeichnet**, daß
das Dihydroxyaceton in Mengenanteilen von 0,5 bis 10 und vorzugsweise von 1 bis 7 Gew.% verwendet wird, bezogen auf das Gesamtgewicht der Zusammensetzung.

3. Zusammensetzung gemäß Anspruch 1 oder 2,
dadurch **gekennzeichnet**, daß
das vernetzte Acrylamid/2-Acrylamido-2-methylpropansulfonsäure-Copolymer ein Copolymer ist, das mit einer olefinisch mehrfach ungesättigten Verbindung vernetzt und mit einem Neutralisiermittel teilweise oder ganz neutralisiert ist, das aus Soda, Pottasche oder einem tertiären Amin ausgewählt ist.

4. Zusammensetzung gemäß einem der Ansprüche 1 bis 3,
dadurch **gekennzeichnet**, daß
das Copolymer aus Acrylamid und 2-Acrylamido-2-methylpropansulfonat durch Copolymerisation von 70 bis 55 Mol% Acrylamid, 30 bis 45 Mol% Natrium-2-acrylamido-2-methylpropansulfonat erhältlich und mit einer olefinisch mehrfach ungesättigten Verbindung mit 10⁻⁴ bis 4 x 10⁻⁴ Mol pro Mol der Mischung der Monomeren vernetzt ist.

5. Zusammensetzung gmeäß einem der Ansprüche 1 bis 4,
dadurch **gekennzeichnet**, daß
das vernetzte Acrylamid/2-Acrylamido-2-methylpropansulfonsäure-Copolymer in einer O/W-Emulsion vorliegt, die 35 bis 40 Gew.% Copolymer, 15 bis 25 Gew.% einer Mischung aus isoparaffinischen C₁₂₋₁₃-Kohlenwasserstoffen, 3 bis 8 Gew.% 7 Mol Ethylenoxid aufweisenden Polyethylenglycollaurylether und Wasser enthält.

6. Zusammensetzung gemäß einem der Ansprüche 1 bis 5,
dadurch **gekennzeichnet**, daß
das teilweise oder ganz neutralisierte Copolymer aus Acrylamid und 2-Acrylamido-2-methylpropansulfonsäure in Mengenanteilen von 0,5 bis 5 und vorzugweise von 0,75 bis 3% Aktivmasse vorhanden ist, bezogen auf das Gesamtgewicht der Zusammensetzung.

7. Zusammensetzung gemäß einem der Ansprüche 1 bis 6,
dadurch **gekennzeichnet**, daß
sie auch fett- und/oder wasserlösliche UV-B- und/oder UV-A-Sonnenfilterstoffe enthält.

8. Zusammensetzung gemäß Anspruch 7,
dadurch **gekennzeichnet**, daß
die Sonnenfilterstoffe aus Zimtsäurederivaten, Salicylaten, Benzylidenkampferderivaten, Bezimidiazolderivaten, Triazinderivaten, Benzophenonderivaten, Dibenzoylmethanderivaten oder aus β,β-Diphenylacrylatderivaten ausgewählt sind.

9. Zusammensetzung gemäß einem der Ansprüche 1 bis 8,
dadurch **gekennzeichnet**, daß
das kosmetisch geeignete Medium Wasser, eine Mischung aus Wasser und einem oder mehreren organischen Lösungsmitteln, ein Lösungsmittel oder eine Mischung aus kosmetisch geeigneten organischen Lösungsmitteln umfaßt.

10. Zusammensetzung gemäß einem der Ansprüche 1 bis 9,
dadurch **gekennzeichnet**, daß
die Zusammensetzung Fettkörper enthält, die aus Ölen und Wachsen mineralischen, tierischen, pflanzlichen und synthetischen Ursprungs, aus Fettsäuren, Fettalkoholen, Triglyceriden von C₆₋₁₈-Fettsäuren und aus Estern von Mono- oder Disäuren ausgewählt sind.

11. Zusammensetzung gemäß einem der Ansprüche 1 bis 10,
dadurch **gekennzeichnet**, daß
sie auch ein Organopolysiloxan enthält.

12. Zusammensetzung gemäß Anspruch 11,
dadurch **gekennzeichnet**, daß
das Organopolysiloxan ein flüchtiges Silicon mit einem Siedepunkt von 60 bis 260°C oder auch ein nicht-flüchtiges Silicon ist, das aus Polyalkylsiloxanen, Polyarylsiloxanen, Polyarylalkylsiloxanen, gegebenenfalls modifizierten Polyethersiloxan-Copolymeren, Gummiprodukten und Harzen aus Silicon und aus organomodifizierten Polysiloxanen sowie aus deren Mischungen ausgewählt ist.

13. Verfahren zur Färbung der Haut, um auf diese eine Bräunung zu übertragen, die der natürlichen Bräunung ähnelt, dadurch **gekennzeichnet**, daß
man auf die Haut mindestens eine in jedem der Ansprüche 1 bis 12 definierte Zusammensetzung aufträgt.
